Europäisches Patentamt

European Patent Office

Office européen des brevets

(11) Numéro de publication : **0 534 812 A1**

# (12) DEMANDE DE BREVET EUROPEEN

(21) Numéro de dépôt : 92402378.1

(22) Date de dépôt : **01.09.92**

(51) Int. Cl.$^5$ : **C07K 3/22,** C07K 13/00

(30) Priorité : **26.09.91 FR 9111849**

(43) Date de publication de la demande :
**31.03.93 Bulletin 93/13**

(84) Etats contractants désignés :
**AT BE CH DE DK ES FR GB GR IE IT LI LU NL PT SE**

(71) Demandeur : **PASTEUR MERIEUX SERUMS ET VACCINS, Société Anonyme :**
**58 Avenue Leclerc**
**F-69007 Lyon (FR)**

(72) Inventeur : **Grandgeorge, Michel**
**12, rue Recret**
**F-69670 Vaugneray (FR)**
Inventeur : **Lutsch, Charles**
**50, Chemin des Cornures**
**F-69290 Grezieu la Varenne (FR)**

(74) Mandataire : **Bernasconi, Jean et al**
**CABINET LEMOINE ET BERNASCONI 13,**
**Boulevard des Batignolles**
**F-75008 Paris (FR)**

(54) **Procédé de purification du facteur VIII et préparations obtenues.**

(57)   Dans le procédé de purification de facteur VIII à partir de cryoprécipité, que l'on dissout, puis que l'on traite au gel d'alumine de façon usuelle,
— l'extrait est dilué à une concentration en protéines inférieure ou égale à 5 g/l environ, notamment 4 g/l, et soumis à une inactivation virale par solvant/détergent,
— puis l'extrait inactivé, contenant le solvant/détergent, est soumis à une chromatographie sur colonne échangeuse d'anions faible, de nature hydrophile,
— le facteur VIII étant ensuite élué par un tampon dissociant.
Préparation obtenue ayant une activité de l'ordre d'au moins 100 UI VIII :C/mg de protéine.

EP 0 534 812 A1

La présente invention a trait à un nouveau procédé de purification du facteur VIII à partir de cryoprécipité de sang humain ou animal, ainsi qu'aux préparations obtenues par ce procédé.

On connaît l'importance qu'il y a à essayer d'obtenir, avec de bons rendements, les fractions les plus pures possible afin d'éviter la présence de contaminants tels qu' autres protéines du sang ou produits étrangers tels qu'antigènes, virus, etc.

Lundblad et al. (Thrombosis Research, 1, p. 197-200, 1972) purifient le facteur VIII bovin par chromatographie échangeuse d'ions sur une colonne de TEAE-cellulose en tampon Tris 50 mM, pH 8,6 et gradient NaCl. Ils obtiennent un rendement de 15 à 45 % et une activité spécifique multipliée par 25 à 50. L'adjonction de 50 mM de glucose dans le tampon augmente le rendement à 60 - 70 % pour une activité spécifique sensiblement identique à la précédente.

Vehar et Davie (Biochemistry, 19 (3), p. 401-410, 1980) purifient le facteur VIII bovin sur DEAE-Sephadex en tampon imidazole 20 mM, à pH 6,6, contenant également : NaCl 0,15 M, CaCl$_2$ 10 mM, glycine éthyl ester 20 mM, glycérine 10 % et dithiotreitol. Le rendement est de 75 % et l'activité spécifique est multipliée par 103.

Faure et al. (Journal of chromatography 257, p. 387-391, 1983) purifient le facteur VIII humain sur un échangeur d'anions à propriétés hydrophobes dues à des résidus hexyles (AH-Sepharose). La solution à purifier est un extrait de cryoprécipité traité au gel d'alumine. En utilisant un tampon d'équilibrage acétate/lysine 0,1 M/0,1 M à pH 5,5, le rendement est de 34 % pour une activité spécifique multipliée par 7,7. L'adjonction à ce tampon de saccharose (10 g/l) et d'albumine (10 g/l) augmenterait le rendement.

Dans la demande de brevet européen EP-A-0 173 242, il est décrit un procédé de purification de facteur VIII, dans lequel un extrait de cryoprécipité traité au gel d'alumine subit une chromatographie sur échangeur d'anions de type polysaccharidique à pH acide compris entre 5 et 6,5, préférentiellement 5,5. Ce procédé est remarquable en ce que, préalablement à la chromatographie, l'extrait de cryoprécipité est pasteurisé et en ce que cette chromatographie s'effectue en présence de saccharose et de glycine en fortes concentrations.

Le brevet US-A-4 743 680 recommande d'une part l'utilisation de sucres et de polyalcools pour augmenter les forces électrostatiques à la surface de la protéine de facteur VIII et diminuer l'hydrophobicité lors de la chromatographie sur colonne échangeuse d'ions et d'autre part l'emploi d'un agent tensio-actif tel que le Tween 80 dans le tampon d'élution de chromatographie. Les supports suggérés sont le QAE-Sephadex A-25 et le QAE-Sepharose 4B - Fast Flow (Pharmacia, Suède).

Le brevet US-A-4 758 657 propose de purifier le facteur VIII sur un support hydrophobe qui n'est pas un échangeur d'anions et d'utiliser un tampon d'élution comprenant des agents tensio-actifs.

D'après la demande de brevet européen EP-A-0 343 275, on connaît également un procédé de purification du facteur VIII dans lequel, principalement, un extrait de cryoprécipité est traité au gel d'alumine à une température comprise entre 10 et 18°C et est soumis à une chromatographie sur colonne d'échange d'anions hydrophile (par exemple sur DEAE-Fractogel). Le procédé comprend une étape d'inactivation virale, à l'aide d'un couple solvant/détergent (par exemple Tween/TNBP) qui peut être ensuite éliminé avant l'étape de chromatographie, par extraction à l'huile. En outre, ce document décrit qu'il est essentiel d'effectuer l'extraction du cryoprécipité dans un milieu contenant de l'héparine. Un traitement similaire est décrit dans la demande de brevet EP-A-0 367 840.

La demande de brevet internationale WO 89/12065 décrit un procédé qui s'inspire sensiblement du précédent (EP-A-0 343 275). Ce document met cependant l'accent sur le choix de la résine de chromatographie de façon à permettre la séparation sur une seule colonne chromatographique et éviter les traitements ultérieurs, tels que l'ultrafiltration, qui, selon cette demande, augmentent la complexité des procédés et diminuent l'activité de la protéine. Le choix de la résine se porte sur un gel de polymère vinylique à groupement DEAE, à propriétés légèrement hydrophobes et ayant une capacité particulière à adsorber les complexes de très grande taille facteur VIII/facteur de Willebrand. Le Fractogel TSK-DEAE 650 (M) (Merck) répond à cette définition et s'avère donc nettement supérieur à d'autres types de gels, tels que le DEAE-Sepharose CL-6B, le DEAE-Sepharose CL-6B Fast-Flow (Pharmacia), le DEAE- Sepharose 4B et le DEAE-Trisacryl LS (IBF), qui sont déconseillés.

Ce document préconise également l'utilisation de glycocolle et de lysine dans le tampon d'élution et évite le recours au chlorure de calcium dans ce tampon. En outre, l'inactivation par solvant/détergent est facultative et peut être mise en oeuvre à un stade quelconque du procédé. Le rendement de chromatographie est compris entre 75 et 90 % et l'activité spécifique du facteur VIII est supérieure à 100 UI/mg.

La présente invention a pour objectif de fournir un nouveau procédé de purification du facteur VIII à partir de cryoprécipité, permettant d'atteindre des rendements de chromatographie supérieurs à 90 %.

Le procédé selon l'invention élimine différents inconvénients de l'art antérieur, et notamment les risques de dénaturation par pasteurisation, l'utilisation de concentrations élevées de sucre, la présence de Tween 80 dans le produit élué ou encore la mise en oeuvre de tampons contenant glycocolle et lysine.

L'invention a donc pour objet un procédé de purification du facteur VIII à partir de cryoprécipité que l'on dissout, puis que l'on traite au gel d'alumine de façon usuelle,

- l'extrait étant alors dilué à une concentration en protéines inférieure ou égale à 5 g/l environ, notamment 4 g/l, et soumis à une inactivation virale par solvant/détergent,
- puis l'extrait inactivé, contenant le solvant/détergent, est soumis à une chromatographie sur colonne échangeuse d'anions faible, de nature hydrophile,
- le facteur VIII étant ensuite élué par un tampon dissociant,
- puis éventuellement soumis à une ultrafiltration, à une filtration stérilisante et à une lyophilisation.

Ce procédé permet d'améliorer le rendement de chromatographie par rapport aux meilleurs procédés connus, tout en garantissant un facteur VIII d'au moins aussi bonne qualité. De façon avantageuse, le procédé selon l'invention permet de se passer des sucres, polyalcools et acides aminés (notamment lysine, glycocolle) utilisés jusqu'à présent dans les étapes de chromatographie.

Les points essentiels de ce procédé qui permettent ces améliorations sont d'une part la limitation du taux de protéine lors de l'étape de chromatographie et d'autre part la présence du solvant/détergent lors de la fixation du facteur VIII sur la colonne, en liaison avec la nature hydrophile de cette dernière.

Le couple solvant/détergent est de préférence choisi parmi les couples suivants :

- Tween 80 / tri-n butyl phosphate (TNBP), notamment 1 % de Tween 80 / 0,3 % de TNBP,
- cholate de sodium / TNBP, notamment 0,2 % de cholate de sodium / 0,3 % de TNBP.

Le support de chromatographie est de préférence de nature polysaccharidique, mais un autre support échangeur d'anions faible, de nature hydrophile et apte à séparer les protéines, peut convenir (par exemple support polyacrylamide-polyvinyle).

Un support particulièrement préféré est le DEAE-Sepharose Fast Flow. Un autre support approprié est le DEAE-Spherodex LS (base dextran/silice).

Les tampons de chromatographie peuvent être avantageusement les suivants :

- tampon d'équilibrage : Tris/HCl 20 mM, NaCl 150 mM, pH 6,8 ;
- tampon de lavage : Tris/HCl 20 mM, NaCl 200 à 210 mM, pH 6,8 ; et
- tampon d'élution : acétate de Na 200 mM, $CaCl_2$ 250 mM, pH 6,0.

L'invention a également pour objet les préparations de facteur VIII obtenues qui se caractérisent par une grande pureté (activité spécifique, avant addition par exemple d'albumine humaine comme stabilisant, d'au moins 100 UI VIII:C/mg de protéine environ).

L'invention va être maintenant décrite plus en détail à l'aide d'exemples de réalisation et d'exemples comparatifs non limitatifs.

**EXEMPLE 1.**

5 kg de cryoprécipité ont été mis en suspension dans 20 l d'eau déminéralisée contenant environ 3 UI/ml d'héparine. 680 ml de gel d'alumine à 2 % ont été ajoutés.

Après incubation à pH neutre et température de +4°C, le mélange a été centrifugé et le surnageant récupéré.

On a obtenu une solution contenant 170.100 UI de facteur VIII avec une activité spécifique de 1,3 UI VIII:C/mg de protéine.

Cette solution a été diluée à 4 g/l de protéines et équilibrée en citrate 20 mM, glycine 100 mM, NaCl 60 mM, $CaCl_2$ 2,5 mM, pH 6,8.

On a procédé ensuite à une inactivation virale par incubation de 15 h à 24°C en présence du mélange solvant/détergent : 0,3 % TNBP + 1 % Tween 80.

Après cette opération, la solution qui contenait 141. 100 UI de facteur VIII a été filtrée à température ambiante sur une colonne de 2,5 l de gel DEAE-Sepharose Fast-Flow prééquilibrée avec le tampon Tris/HCl 20 mM, NaCl 150 mM, pH 6,8.

Après lavage de la colonne par un tampon Tris/HCl 20 mM; NaCl 210 mM, pH 6,8, le facteur VIII a été élué par un tampon acétate de sodium 200 mM, $CaCl_2$ 250 mM, pH 6,8.

On a obtenu une solution contenant 135.600 UI de facteur VIII purifié avec une activité spécifique de 141,2 UI VIII:C/mg de protéine, soit un rendement chromatographique de 96,1 %.

A ce stade, le facteur VIII a été débarrassé de l'excédent de $CaCl_2$ et équilibré dans une solution stabilisante, compatible avec une utilisation intraveineuse, par diafiltration, puis lyophilisé.

La solution stabilisante utilisée dans cet exemple contenait de l'albumine humaine, de sorte que l'activité spécifique finale a été abaissée à 6,6 UI VIII:C/mg de protéine.

**EXEMPLE 2.**

**Influence de la concentration protéique sur le pourcentage de fixation sur la colonne.**

Les essais ci-après ont été réalisés sur une colonne de 145 ml de gel DEAE-Sepharose Fast-Flow. Les solutions de facteur VIII purifiées au gel d'alumine ont été préparées comme dans l'exemple 1, sauf l'ajustement en protéines qui a été variable.

Le mélange solvant/détergent utilisé pour l'inactivation virale a été soit 0,3 % TNBP + 1 % Tween 80, soit 0,3 % TNBP + 0,2 % cholate de sodium. L'injection sur la colonne, équilibrée comme dans l'exemple 1, s'est effectuée à température ambiante à un débit de 1 l/h.

Le pourcentage de fixation du facteur VIII sur la colonne a été calculé à partir des unités VIII:C non retenues sur la colonne (unités retrouvées dans le filtrat).

Les résultats reportés dans le tableau 1 montrent que le pourcentage de fixation de facteur VIII sur la colonne est abaissé pour une concentration protéique > 5 g/l. A une concentration protéique < 5 g/l, le pourcentage de fixation est toujours > 95 % pour une quantité de facteur VIII comprise entre 3.700 UI VIII:C et 11.200 UI VIII:C.

TABLEAU 1.

| N° essai | Solvant/ Détergent | Concentra- tion pro- téique g/l | Unités VIII:C injectées | % de fixation |
|---|---|---|---|---|
| 622-44 | TNBP/Tween | 11,2 | 5 467 | 82,3 |
| 622-43 | TNBP/Tween | 4,7 | 11 200 | 96,3 |
| 622-45 | TNBP/Tween | 4,6 | 4 952 | 95,3 |
| 622-58 | TNBP/Tween | 3,1 | 3 773 | 98,5 |
| 622-50 | TNBP/cholate | 7,3 | 7 665 | 82,2 |
| 622-51 | TNBP/cholate | 6,0 | 7 592 | 90,8 |
| 622-47 | TNBP/cholate | 3,9 | 6 351 | 95,7 |
| 622-41 | TNBP/cholate | 3,7 | 6 716 | 96,3 |

**EXEMPLE 3**

**Comparaison des supports chromatographiques DEAE-Sepharose Fast-Flow (Pharmacia) et DEAE-Spherodex LS (IBF).**

Ces essais ont été réalisés sur une colonne de 20 ml de gel. Une même solution de facteur VIII purifiée au gel d'alumine a été préparée selon un procédé analogue à celui décrit dans l'exemple 1, puis soumise à un traitement d'inactivation virale par le mélange solvant/détergent 0,3 % TNBP + 1 % Tween 80. Le facteur VIII avait une activité spécifique à ce stade de 1,0 UI VIII:C/mg de protéine.

La chromatographie a été effectuée dans des conditions similaires à l'exemple 1, comparativement sur support DEAE-Sepharose et DEAE-Spherodex. Le rendement de chromatographie a été calculé à partir des unités de facteur VIII retrouvées dans l'éluat.

Les résultats reportés dans le tableau 2 montrent des performances identiques pour les deux supports chromatographiques.

## TABLEAU 2.

| N° essai | Support chromatographique | Unités VIII:C injectées | Rendement de chromatographie | Activité spécifique du facteur VIII dans l'éluat (UI VIII:C/mg protéine) |
|---|---|---|---|---|
| 622-18 | DEAE-Spherodex | 116 | 92,1 % | 113 |
| 622-21 | DEAE-Sepharose | 110 | 93,0 % | 115 |

## EXEMPLE 4.

**Conséquence de l'absence du réactif solvant/détergent dans le facteur VIII sur la purification chromatographique.**

Cet essai a été réalisé sur une colonne de 20 ml de gel DEAE-Sepharose Fast-Flow à partir de solution de facteur VIII purifiée au gel d'alumine selon un procédé analogue à celui décrit dans l'exemple 1. Le facteur VIII avait une activité spécifique à ce stade de 1,0 UI VIII:C/mg de protéine.

Le produit a été chromatographié directement, sans effectuer de traitement solvant/détergent préalable, dans des conditions identiques à l'essai 622-21 de l'exemple 3.

Les résultats rapportés dans le tableau 3 sont moins satisfaisants que ceux obtenus dans l'essai 622-21, malgré un taux de protéines à l'injection plus favorable (4,5 g/l contre 6,0 g/l dans l'essai 622-21).

## TABLEAU 3.

| N° essai | Traitement solvant/détergent | Unités VIII:C injectées | Rendement de chromatographie | Activité spécifique du facteur VIII dans l'éluat (UI VIII:C/mg protéine) |
|---|---|---|---|---|
| 622-24 | NON | 93 | 80 % | 70 |

## Revendications

1. Procédé de purification de facteur VIII à partir de cryoprécipité, que l'on dissout, puis que l'on traite au gel d'alumine de façon usuelle,
   - l'extrait étant dilué à une concentration en protéines inférieure ou égale à 5 g/l environ, notamment 4 g/l, et soumis à une inactivation virale par solvant/détergent,
   - puis l'extrait inactivé, contenant le solvant/détergent, est soumis à une chromatographie sur colonne échangeuse d'anions faible, de nature hydrophile,
   - le facteur VIII étant ensuite élué par un tampon dissociant.

2. Procédé selon la revendication 1, caractérisé en ce que le facteur VIII élué est soumis à des opérations d'ultrafiltration, de filtration stérilisante et de lyophilisation.

3. Procédé selon l'une des revendications 1 et 2, caractérisé en ce que l'on utilise un couple solvant/ détergent choisi parmi les couples suivants :
   - Tween 80/TNBP, notamment 1% de Tween 80/0,3 % de TNBP,
   - cholate de sodium/TNBP, notamment 0,2 % de cholate de sodium/0,3 % de TNBP.

4. Procédé selon l'une des revendications 1 à 3, caractérisé en ce que le support de chromatographie est un support polysaccharidique.

5. Procédé selon la revendication 4, caractérisé en ce que l'on utilise comme support de chromatographie le DEAE-Sepharose Fast-Flow ou le DEAE-Spherodex LS.

6. Procédé selon l'une des revendications 1 à 5, caractérisé en ce que les tampons de chromatographie utilisés sont choisis parmi certains ou la totalité des tampons suivants :
- tampon d'équilibrage : Tris/HCl 20 mM, NaCl 150 mM, pH 6,8 ;
- tampon de lavage : Tris/HCl 20 mM, NaCl 200 à 210 mM, pH 6,8 ; et
- tampon d'élution : acétate de Na 200 mM, $CaCl_2$ 250 mM, pH 6,0.

7. Préparation de facteur VIII obtenue par le procédé selon l'une quelconque des revendications 1 à 6, ayant une activité de l'ordre d'au moins 100 UI VIII:C/mg de protéine, avant addition éventuelle d'un stabilisant, tel que de l'albumine humaine.

**Office européen des brevets**

# RAPPORT DE RECHERCHE EUROPEENNE

Numero de la demande

EP 92 40 2378

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 367 840 (OCTAPHARMA AG) * page 3, colonne 3, ligne 21 - page 3, colonne 4, ligne 12; revendications 1-10; exemples 2-5 * | 1-7 | C07K3/22 C07K13/00 |

-----

**DOMAINES TECHNIQUES RECHERCHES (Int. Cl.5)**

C07K
A61K

Le présent rapport a été établi pour toutes les revendications

| Lieu de la recherche | Date d'achèvement de la recherche | Examinateur |
|---|---|---|
| LA HAYE | 14 DECEMBRE 1992 | RYCKEBOSCH A.O. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)